# EUROPEAN PATENT SPECIFICATION

(11) **EP 3 065 565 B1**
(45) Date of publication and mention of the grant of the patent: **25.12.2019**
(21) Application number: 14798759.8
(22) Date of filing: 06.11.2014
(51) Int. Cl.: A23L 33/10, A61K 9/16, C07C 403/24

(54) **PROCESS FOR THE PURIFICATION OF ASTAXANTHIN**
VERFAHREN ZUR REINIGUNG VON ASTAXANTHIN
PROCESSUS DE PURIFICATION D'ASTAXANTHINE

(30) Priority: 07.11.2013 US 201361901136 P; 12.11.2013 EP 13192491
(43) Date of publication of application: 14.09.2016
(60) Divisional application: 19160438.8
(73) Proprietor: DSM IP Assets B.V., 6411 TE Heerlen (NL)
(72) Inventor: KARRER, Philippe, 4303 Kaiseraugst (CH); WUESTENBERG, Bettina, 4303 Kaiseraugst (CH)
(74) Representative: Steck, Melanie
(86) International application number: PCT/EP2014/073965
(87) International publication number: WO 2015/067711

(56) References cited:
- EP-A1- 0 633 258
- WO-A1-01/19383
- WO-A1-2007/128574
- WO-A1-2009/144329
- WO-A1-2010/058235
- WO-A2-2007/072529
- CN-A- 103 044 304
- AMBATI RANGA RAO ET AL: "Stabilization of astaxanthin in edible oils and its use as an antioxidant", JOURNAL OF THE SCIENCE OF FOOD AND AGRICULTURE, vol. 87, no. 6, 30 April 2007 (2007-04-30) , pages 957-965, XP055120279, ISSN: 0022-5142, DOI: 10.1002/jsfa.2766

## Description

The object of the invention is to provide a synthetic food-grade astaxanthin (AXN) which is not known so far. "Food-grade" within the present invention means "suitable for human consumption". Such a food-grade synthetic AXN thus fulfills all regulatory requirements concerning purity and can be used in the commercial production of food and dietary supplements.

AXN can be manufactured by chemical synthesis (see EP-A 908 449, WO 2005/087720, EP-A 733 619), it can be produced by fermentation (see EP-A 543 023) and isolated from natural sources such as shell waste (see JP-A 11-049 972) or algae *Haematococcus pluvialis* (see GB-A 2,301,587), where the isolated esters would have to be cleaved to AXN itself.

AXN isolated from natural sources is already used in products for human consumption: see e.g. US 2009/0297492, where AXN is described as improving the cognitive performance; US 2009/0018210, where AXN is described as promoting fat degradation; EP-A 1 867 327, where AXN is described for preventing a neurodegenerative disease - to name only a few. So far, however, synthetic AXN was not used in products for human consumption.

Synthetic AXN is of standardized quality compared to AXN from natural sources since it is much easier to use a standardized procedure for its chemical synthesis and purification than being dependent from the varying quality of natural AXN resulting by using natural sources which vary also in their composition. Thus, in the context of the present invention the term "AXN" means "synthetic AXN".

Chemical processes for the manufacture of AXN are often carried out in halogenated hydrocarbons such as dichloromethane (see e.g. WO 2011/095571) since AXN and its precursors have a high solubility in these solvents though there were intentions in the past to avoid the use of such solvents (see e.g. EP-A 908 449 and WO 2005/087720). There are also purification methods described that use chloroform (see JP-A 07118226). The thus obtained AXN is then often crystallized from lower alkanols since it is hardly soluble in such solvents.

There exist already processes for the purification of astaxanthin: In example 11 of WO 2007/072529 the synthesis of astaxanthin is performed in isopropanol. The obtained product is recrystallized in three successive rounds: one with methylene chloride, one with methanol and one with heptane. Hereby all astaxanthin is dissolved. According to WO 2007/128574 (see page 9, line 25) the filter cake of astaxanthin is washed with methanol. According to EP-A 633 258 (see example 2) the astaxanthin crystals have been washed with heptane.

It was therefore an object of the present invention to provide a synthetic AXN which is of reliable quality, easy to obtain also in an industrial scale and suitable for human consumption.

Thus, it was an object of the present invention to provide a food-grade synthetic AXN. This object is met by the process of the present invention which yields an AXN with extremely low solvent levels, preferably with extremely low levels of dichloromethane, thus rendering this AXN suitable for human consumption.

Thus, the present invention is especially directed to the synthesis of a synthetic AXN with a content of dichloromethane ≤ 250 ppm, preferably with a content of dichloromethane ≤ 200 ppm, more preferably with a content of dichloromethane ≤ 100 ppm, even more preferably with a content of dichloromethane ≤ 50 ppm, ≤ 35 ppm, ≤ 30 ppm, ≤ 25 ppm, ≤ 20 ppm, most preferably with a content of dichloromethane ≤ 10 ppm.

In an especially preferred embodiment of the present invention the synthetic AXN has a content of dichloromethane in the range of between 0 and 100 ppm, preferably in the range of between 10 and 100 ppm.

Preferably the synthetic AXN prepared according to the present invention has a content of methanol ≤ 500 ppm, preferably it has a content of methanol ≤ 350 ppm, more preferably it has a content of methanol ≤ 250 ppm, even more preferably it has a content of methanol ≤ 150 ppm, ≤ 100 ppm, most preferably it has a content of methanol ≤ 50 ppm, ≤ 20 ppm, ≤ 10 ppm, ≤ 5 ppm.

In an especially preferred embodiment of the present invention the synthetic AXN has a content of methanol in the range of between 0 and 50 ppm, preferably in the range of between 0 and 20 ppm, more preferably in the range of 0 to 10 ppm, most preferably in the range of 0 to 5 ppm.

In a further preferred embodiment the synthetic AXN has a content of dichloromethane < 50 ppm and a content of methanol < 100 ppm.

The synthetic AXN prepared according to the present invention may especially be used in dietary supplements. For these purposes it is often provided in the form of an oily suspension or a powdery formulation such as a beadlet, thus protecting the synthetic AXN from degradation.

Since it is possible with the processes of the present invention to significantly reduce the content of halogenated organic solvents such as dichloromethane, there is no need any more to use a chemical synthesis, where the use of such solvents is avoided.

### Processes

Such an AXN may be obtained by one of the following processes which are objects of the present invention.

### Process A

A process for the manufacture of synthetic astaxanthin suitable for human consumption comprising the following steps:
a) Providing synthetic astaxanthin crystals containing dichloromethane; and
b) Adding methanol to the synthetic astaxanthin crystals in an amount to obtain a suspension of synthetic astaxanthin in methanol, whereby the amount of synthetic astaxanthin in said suspension is in the range of from 5 to 70 weight-%, based on the total weight of the suspension; and
c) Heating up the suspension obtained in step b) to a temperature in the range of from 80 to 140°C in a closed reactor; and
d) Maintaining the suspension at a temperature in the range as given for step c) until the content of dichloromethane in the synthetic astaxanthin is ≤ 250 ppm; and
e) Cooling the methanolic synthetic astaxanthin suspension, whereby its content of dichloromethane is ≤ 250 ppm, to a temperature in the range of from 10 to 35°C; and
f) Filtering off the synthetic astaxanthin crystals and optionally drying them; whereby a synthetic astaxanthin suitable for human consumption is obtained.

### Process B

A process for the manufacture of synthetic astaxanthin suitable for human consumption (starting material: solution of synthetic astaxanthin in dichloromethane) comprising the following steps:
a1) Providing a solution of synthetic astaxanthin in dichloromethane; and
a2) optionally adding methanol to the synthetic astaxanthin solution of step a1); and
a3) removing dichloromethane and optionally methanol by distillation; and
b1) Adding methanol to the synthetic astaxanthin solution in an amount to obtain a suspension of synthetic astaxanthin, whereby the amount of synthetic astaxanthin in said suspension is in the range of from 5 to 70 weight-%, based on the total weight of the suspension; and
c1) Heating up the suspension obtained in step b1) to a temperature in the range of from 80 to 140°C in a closed reactor; and
d1) Maintaining the suspension at a temperature in the range as given for step c1) until the content of dichloromethane in the synthetic astaxanthin is ≤ 250 ppm; and
e1) Cooling the methanolic synthetic astaxanthin suspension, whereby its content of dichloromethane is ≤ 250 ppm, to a temperature in the range of from 10 to 35°C; and
f1) Filtering off the synthetic astaxanthin crystals and optionally drying them; whereby a synthetic astaxanthin suitable for human consumption is obtained.

These processes will now be described in more detail below. They are economic and may be performed in an industrial scale. During the processes according to the present invention the total amount of synthetic astaxanthin is not completely dissolved in the methanol. This is in contrast to a crystallization process where the total amount of synthetic astaxanthin is completely dissolved upon heating and re-crystallizes when the temperature is decreased again.

Since the steps of Process A and B only differ with respect to the starting material, they are the same and thus, described together.

### Step a)/step a1)

As starting material either synthetic AXN crystals (see process A - step a)) or a solution of synthetic AXN in dichloromethane (see process B - step a1)) may be used.
Process A: The synthetic AXN crystals are as obtained from any of the chemical syntheses after removing of the solvent, in which the synthesis has been performed as e.g. preferably in dichloromethane. The dichloromethane may have been removed by distillation or by solvent exchange. Preferably the dichloromethane has been removed by a solvent exchange with methanol. In this case the mixture of astaxanthin and dichloromethane is heated up to a temperature of 38 to 40°C at atmospheric pressure and dichloromethane is distilled off. Simultaneously methanol is added so that the volume of the mixture is kept constant. Then solvent (dichloromethane + methanol) are distilled off until the internal temperature has been raised to 64°C (boiling point of methanol) meaning that only methanol is distilled off, but no dichloromethane any more.
   These synthetic AXN crystals still contain dichloromethane which cannot be removed by just prolonging the time for drying these synthetic AXN crystals, even if the temperature and/or the vacuum is/are increased. Typical amounts of remaining dichloromethane in the synthetic AXN crystals are 0.2 to 0.3 weight-%, based on the total weight of the synthetic AXN crystals.
Process B: Since the process of the present invention is very effective in removing dichloromethane, it is also possible to use directly a solution of the synthetic AXN in dichloromethane. In this case it is advantageous to first reduce the amount of dichloromethane in the solution by distillation. Preferably this is done whereby simultaneously methanol is added.

Thus, if process B is performed advantageously steps a2) and a3) are also performed.
a2) adding methanol to the synthetic astaxanthin solution of step a1);
a3) removing dichloromethane and methanol by distillation.

### Step b)/step b1)

Preferably step b)/step b1) is carried out in a closed reactor.

Preferably the methanol is added to the synthetic astaxanthin crystals (step b) - Process A) or the solution of synthetic AXN in dichloromethane (step b1) - Process B) in an amount to obtain a suspension of synthetic astaxanthin in methanol, whereby the amount of synthetic astaxanthin in said suspension is in the range of from 20 to 60 weight-%, more preferably in the range of from 30 to 50 weight-%. Preferably this is done under nitrogen.

### Step c)/Step c1)

Preferably the suspension obtained in step b)/step b1) is heated up to a temperature, i.e. brought to a temperature, in the range of from 80 to 140°C, more preferably to a temperature in the range of from 90 to 125°C, even more preferably to a temperature in the range of from 100 to 120°C, most preferably to a temperature in the range of from 105 to 115°C, in a closed reactor. Thereby the pressure increases.

### Step d)/step d1)

The temperature is maintained constant at the wanted value - the same temperature as in step c)/c1) - until the content of dichloromethane in the synthetic astaxanthin is ≤ 250 ppm, preferably ≤ 200 ppm, more preferably ≤ 100 ppm, even more preferably ≤ 50 ppm, ≤ 35 ppm, ≤ 30 ppm, ≤ 25 ppm, ≤ 20 ppm, most preferably ≤ 10 ppm.

### Step e)/step e1)

Preferably the methanolic synthetic astaxanthin suspension, whereby its content of dichloromethane is ≤ 250 ppm, is cooled to a temperature in the range of from 15-30°C, more preferably to a temperature in the range of from 20 to 25°C.

### Step f)/step f1)

The synthetic astaxanthin crystals are then filtered off and optionally dried.

The drying of the obtained synthetic AXN crystals is generally carried out at a temperature below 140°C, preferably at a temperature in the range of from 40 to 140°C, and in vacuum. The vacuum is preferably in a range of between 0 and 20 mbara.

In one embodiment of the present invention the drying of the synthetic AXN crystals is carried out at a temperature of 80°C and at 20 mbar. In a further embodiment of the present invention the drying of the synthetic AXN crystals is carried out at a temperature in the range of from 55 to 70°C and at a pressure below 20 mbar.

An embodiment of the present invention is a process, where the steps a) to f) and a1) to f1), respectively, are carried out twice, meaning that the astaxanthin obtained in step f) or in step f1), respectively, is used as starting material for step a) and step a1), respectively.

Especially preferred embodiments of the present invention are those processes, whereby one or more of the preferred conditions given above are realized. The most preferred processes of the present invention are those, whereby all preferred conditions given above are realized.

The invention is now further illustrated in the following non-limiting example.

### Examples

### Example 1: Process for obtaining synthetic food-grade astaxanthin

120 g of astaxanthin (97%) containing 2000 mg/kg of dichloromethane are introduced in a 1000 ml steel autoclave under inert conditions with 280 ml of methanol. The autoclave is closed, the mixture agitated at 350 rpm (rotations per minute) and heated at 105°C (internal temperature). Once the wanted internal temperature (105°C) is reached, the mixture is kept constantly at this temperature for 7 hours under stirring. After 7 hours of this treatment, the mixture is cooled to 25°C and diluted with 50 ml methanol. The crystals are filtered off, washed with 150 ml of methanol and dried in a drying oven 16 hours at 80°C under 20 mbara. Dry astaxanthin containing 45 mg/kg of dichloromethane is obtained.

In principle is it possible to perform example 1 as described above also at any other temperature in the range of from 80 to 140°C instead of 105°C. Such other temperatures are e.g. 80°C, 85°C, 90°C, 95°C, 100°C, 110°C, 115°C, 120°C, 125°C, 130°C, 135°C, 140°C and any other temperature in between. Example 1 may especially be repeated at a temperature of 110°C and 115°C, respectively.

## Claims

1. A process for the manufacture of synthetic food-grade astaxanthin comprising the following steps:
a) Providing synthetic astaxanthin crystals containing dichloromethane; and
b) Adding methanol to the synthetic astaxanthin crystals in an amount to obtain a suspension of synthetic astaxanthin in methanol, whereby the amount of synthetic astaxanthin in said suspension is in the range of from 5 to 70 weight-%, based on the total weight of the suspension; and
c) Heating up the suspension obtained in step b) to a temperature in the range of from 80 to 140°C in a closed reactor; and
d) Maintaining the suspension at a temperature in the range as given for step c) until the content of dichloromethane in the synthetic astaxanthin is ≤ 250 ppm; and
e) Cooling the methanolic synthetic astaxanthin suspension, whereby its content of dichloromethane is ≤ 250 ppm, to a temperature in the range of from 10 to 35°C; and
f) Filtering off the synthetic astaxanthin crystals and optionally drying them;
whereby a synthetic food-grade astaxanthin is obtained.

2. A process for the manufacture of synthetic food-grade astaxanthin comprising the following steps:
a1) Providing a solution of synthetic astaxanthin in dichloromethane; and
a2) optionally adding methanol to the synthetic astaxanthin solution of step a1); and
a3) removing methanol, if present, and dichloromethane by distillation; and
b1) Adding methanol to the synthetic astaxanthin solution in an amount to obtain a suspension of synthetic astaxanthin, whereby the amount of synthetic astaxanthin in said suspension is in the range of from 5 to 70 weight-%, based on the total weight of the suspension; and
c1) Heating up the suspension obtained in step b1) to a temperature in the range of from 80 to 140°C in a closed reactor; and
d1) Maintaining the suspension at a temperature in the range as given for step c1) until the content of dichloromethane in the synthetic astaxanthin is ≤ 250 ppm; and
e1) Cooling the methanolic synthetic astaxanthin suspension, whereby its content of dichloromethane is ≤ 250 ppm, to a temperature in the range of from 10 to 35°C; and
f1) Filtering off the synthetic astaxanthin crystals and optionally drying them; whereby a synthetic food-grade astaxanthin is obtained.

3. The process according to claim 1 or 2, wherein steps b) and e) or steps b1) and e1), respectively, are carried out simultaneously.

4. The process according to claim 1 or 2, whereby in step b) or in step b1) the amount of synthetic astaxanthin in said suspension is in the range of from 20 to 60 weight-%, preferably the amount of synthetic astaxanthin in said suspension is in the range of from 30 to 50 weight-%, based on the total weight of the suspension.

5. The process according to claim 1 or 2 or 4, whereby in step c) or in step c1) the suspension is brought to a temperature in the range of from 90 to 125°C, preferably to a temperature in the range of from 100 to 120°C, more preferably to a temperature in the range of from 105 to 115°C; and in step d) or in step d1) the suspension is then maintained at said temperature.

6. The process according to claim 1 or 2 or 4 or 5, whereby in step e) or in step e1) the methanolic synthetic AXN suspension is cooled to a temperature in the range of from 15 to 30°C, preferably to a temperature in the range of from 20 to 25°C.

## Patentansprüche

1. Verfahren zur Herstellung von synthetischem Astaxanthin in Lebensmittelqualität, das folgende Schritte umfasst:
a) Bereitstellen von Kristallen von synthetischem Astaxanthin, die Dichlormethan enthalten; und
b) Zugeben von Methanol zu den Kristallen von synthetischem Astaxanthin in einer Menge zum Erhalt einer Suspension von synthetischem Astaxanthin in Methanol, wobei die Menge von synthetischem Astaxanthin in der Suspension im Bereich von 5 bis 70 Gew.-%, bezogen auf das Gesamtgewicht der Suspension, liegt; und
c) Erhitzen der in Schritt b) erhaltenen Suspension auf eine Temperatur im Bereich von 80 bis 140°C in einem geschlossenen Reaktor; und
d) Halten der Suspension bei einer Temperatur in dem für Schritt c) angegebenen Bereich, bis der Gehalt an Dichlormethan in dem synthetischen Astaxanthin ≤ 250 ppm ist; und
e) Abkühlen der methanolischen Suspension von synthetischem Astaxanthin, wobei der Dichlormethan-Gehalt der Suspension ≤ 250 ppm ist, auf eine Temperatur im Bereich von 10 bis 35°C; und
f) Abfiltrieren der Kristalle von synthetischem Astaxanthin und gegebenenfalls Trocknen der Kristalle; wodurch ein synthetisches Astaxanthin in Lebensmittelqualität erhalten wird.

2. Verfahren zur Herstellung von synthetischem Astaxanthin in Lebensmittelqualität, das folgende Schritte umfasst:
a1) Bereitstellen einer Lösung von synthetischem Astaxanthin in Dichlormethan; und
a2) gegebenenfalls Zugeben von Methanol zu der Lösung von synthetischem Astaxanthin aus Schritt a1); und
a3) Entfernen von Methanol, sofern vorhanden, und Dichlormethan durch Destillation; und
b1) Zugeben von Methanol zu der Lösung von synthetischem Astaxanthin in einer Menge zum Erhalt einer Suspension von synthetischem Astaxanthin in Methanol, wobei die Menge von synthetischem Astaxanthin in der Suspension im Bereich von 5 bis 70 Gew.-%, bezogen auf das Gesamtgewicht der Suspension, liegt; und
c1) Erhitzen der in Schritt b1) erhaltenen Suspension auf eine Temperatur im Bereich von 80 bis 140°C in einem geschlossenen Reaktor; und
d1) Halten der Suspension bei einer Temperatur in dem für Schritt c1) angegebenen Bereich, bis der Gehalt an Dichlormethan in dem synthetischen Astaxanthin ≤ 250 ppm ist; und
e1) Abkühlen der methanolischen Suspension von synthetischem Astaxanthin, wobei der Dichlormethan-Gehalt der Suspension ≤ 250 ppm ist, auf eine Temperatur im Bereich von 10 bis 35°C; und
f1) Abfiltrieren der Kristalle von synthetischem Astaxanthin und gegebenenfalls Trocknen der Kristalle; wodurch ein synthetisches Astaxanthin in Lebensmittelqualität erhalten wird.

3. Verfahren nach Anspruch 1 oder 2, bei dem die Schritte b) und e) bzw. die Schritte b1) und e1) gleichzeitig durchgeführt werden.

4. Verfahren nach Anspruch 1 oder 2, wobei in Schritt b) bzw. in Schritt b1) die Menge von synthetischem Astaxanthin in der Suspension im Bereich von 20 bis 60 Gew.-% liegt, vorzugsweise die Menge von synthetischem Astaxanthin in der Suspension im Bereich von 30 bis 50 Gew.-% liegt, bezogen auf das Gesamtgewicht der Suspension.

5. Verfahren nach Anspruch 1 oder 2 oder 4, wobei in Schritt c) bzw. in Schritt c1) die Suspension auf eine Temperatur im Bereich von 90 bis 125°C, vorzugsweise auf eine Temperatur im Bereich von 100 bis 120°C und weiter bevorzugt auf eine Temperatur im Bereich von 105 bis 115°C gebracht wird und in Schritt d) bzw. in Schritt d1) die Suspension dann bei dieser Temperatur gehalten wird.

6. Verfahren nach Anspruch 1 oder 2 oder 4 oder 5, wobei in Schritt e) bzw. in Schritt e1) die methanolische Suspension von synthetischem AXN auf eine Temperatur im Bereich von 15 bis 30°C und vorzugsweise auf eine Temperatur im Bereich von 20 bis 25°C abgekühlt wird.

## Revendications

1. Procédé pour la préparation d'astaxanthine synthétique de qualité alimentaire comprenant les étapes suivantes :
a) la mise à disposition de cristaux d'astaxanthine synthétique contenant du dichlorométhane ; et
b) l'ajout de méthanol aux cristaux d'astaxanthine synthétique en une quantité pour obtenir une suspension d'astaxanthine synthétique dans du méthanol, la quantité d'astaxanthine synthétique dans ladite suspension se situant dans la plage de 5 jusqu'à 70% en poids, sur la base du poids total de la suspension ; et
c) le chauffage de la suspension obtenue dans l'étape b) à une température dans la plage de 80 jusqu'à 140°C dans un réacteur clos ; et
d) le maintien de la suspension à une température dans la plage telle que mentionnée pour l'étape c) jusqu'à ce que la teneur en dichlorométhane dans l'astaxanthine synthétique soit ≤ 250 ppm ; et
e) le refroidissement de la suspension méthanolique d'astaxanthine synthétique, dont la teneur en dichlorométhane est ≤ 250 ppm, à une température dans la plage de 10 jusqu'à 35°C ; et
f) la filtration des cristaux d'astaxanthine synthétique et éventuellement le séchage de ceux-ci ;
par lequel une astaxanthine synthétique de qualité alimentaire est obtenue.

2. Procédé pour la préparation d'astaxanthine synthétique de qualité alimentaire comprenant les étapes suivantes :
a1) la mise à disposition d'une solution d'astaxanthine synthétique dans du dichlorométhane ; et
a2) éventuellement, l'ajout de méthanol à la solution d'astaxanthine synthétique de l'étape a1) ;
et
a3) l'élimination de méthanol, le cas échéant, et de dichlorométhane par distillation ; et
b1) l'ajout de méthanol à la solution d'astaxanthine synthétique en une quantité pour obtenir une suspension d'astaxanthine synthétique, la quantité d'astaxanthine synthétique dans ladite suspension se situant dans la plage de 5 jusqu'à 70% en poids, sur la base du poids total de la suspension ; et
c1) le chauffage de la suspension obtenue dans l'étape b1) à une température dans la plage de 80 jusqu'à 140°C dans un réacteur clos ; et
d1) le maintien de la suspension à une température dans la plage telle que mentionnée pour l'étape c1) jusqu'à ce que la teneur en dichlorométhane dans l'astaxanthine synthétique soit ≤ 250 ppm ;
et
e1) le refroidissement de la suspension méthanolique d'astaxanthine synthétique, dont la teneur en dichlorométhane est ≤ 250 ppm, à une température dans la plage de 10 jusqu'à 35°C; et f1) la filtration des cristaux d'astaxanthine synthétique et éventuellement le séchage de ceux-ci ;
par lequel une astaxanthine synthétique de qualité alimentaire est obtenue.

3. Procédé selon la revendication 1 ou 2, les étapes b) et e) ou les étapes b1) et e1), respectivement, étant mises en œuvre simultanément.

4. Procédé selon la revendication 1 ou 2, dans lequel dans l'étape b) ou dans l'étape b1) la quantité d'astaxanthine synthétique dans ladite suspension se situe dans la plage de 20 jusqu'à 60% en poids, préférablement la quantité d'astaxanthine synthétique dans ladite suspension se situe dans la plage de 30 jusqu'à 50% en poids, sur la base du poids total de la suspension.

5. Procédé selon la revendication 1 ou 2 ou 4, dans lequel dans l'étape c) ou dans l'étape c1) la suspension est amenée à une température dans la plage de 90 jusqu'à 125°C, préférablement à une température dans la plage de 100 jusqu'à 120°C, plus préférablement à une température dans la plage de 105 jusqu'à 115°C ; et dans l'étape d) ou dans l'étape d1) la suspension est ensuite maintenue à ladite température.

6. Procédé selon la revendication 1 ou 2 ou 4 ou 5, dans lequel dans l'étape e) ou dans l'étape e1) la suspension méthanolique d'AXN (astaxanthine) synthétique est refroidie à une température dans la plage de 15 jusqu'à 30°C, préférablement à une température dans la plage de 20 jusqu'à 25°C.
